# EUROPEAN PATENT APPLICATION

(11) **EP 4 159 764 A1**
(43) Date of publication of application: **05.04.2023**
(21) Application number: 21817549.5
(22) Date of filing: 01.06.2021
(51) Int. Cl.: C07K 16/46, C12N 15/13, A61K 39/395, A61P 35/00

(54) **ANTI-PDL1×EGFR BISPECIFIC ANTIBODY**

(30) Priority: 02.06.2020 CN 202010487524
(71) Applicant: Zeda Biopharmaceuticals, Inc., Tortola, VG 1110 (VG)
(72) Inventor: ZHU, Zhenping, Shanghai 201203 (CN); HUANG, Haomin, Shanghai 201203 (CN); GU, Changling, Shanghai 201203 (CN); ZHU, Haixia, Shanghai 201203 (CN)
(74) Representative: reuteler & cie SA
(86) International application number: PCT/CN2021/097785
(87) International publication number: WO 2021/244554

(57) **Abstract**

Provided is an anti-PDLl × EGFR bispecific antibody. Experimental results show that the bispecific antibody can maintain relatively well the activity of anti-EGFR and anti-PD-L1 monoclonal antibodies; in addition, same can specifically bind to the two PD-Ll and EGFR targets at the same time, and more importantly, said bispecific antibody has obvious synergy at the cellular level.

## Description

### Technical field

The present invention relates to the field of antibodies, in particular to an anti-PDL1 × EGFR bispecific antibody.

### Background

Human Programmed Cell Death receptor-1 (PD-1) is a type I membrane protein with 288 amino acids. It is one of the major known immune checkpoints (Blank et al, 2005, Cancer Immunotherapy, 54:307-314). PD-1 is expressed in already activated T lymphocytes and the binding of PD-1 to its ligands PD-L1 (programmed cell death-Ligand 1) and PD-L2 (programmed cell death-Ligand 2) can inhibit the activity of T lymphocytes and the associated cellular immune response *in vivo.* PD-L2 is mainly expressed in macrophages and dendritic cells, while PD-L1 is widely expressed in B lymphocytes, T lymphocytes and peripheral cells such as microvascular epithelial cells, lung, liver, heart and other tissue cells. A large number of studies have shown that the interaction between PD-1 and PD-L1 is not only necessary to maintain the balance of the immune system *in vivo,* but also the main mechanism and reason causing PD-L1 positive tumor cells to evade immune surveillance. By blocking the negative regulation of the PD-1/PD-L1 signaling pathway by cancer cells and activating the immune system, it can promote the tumor-specific cellular immune response associated with T cells, thus opening the door to a new tumor therapy approach -- tumor immunotherapy.

PD-1 (encoded by the gene Pdcd1) is a member of the immunoglobulin superfamily associated with CD28 and CTLA-4. Studies have shown that PD-1 negatively regulates antigen receptor signal transduction when it binds to its ligands (PD-L1 and/or PD-L2). At present, the structure of mouse PD-1 and the co-crystallization structure of mouse PD-1 and human PD-L1 have been clarified (Zhang, X. et al., Immunity 20:337-347 (2004); Lin et al., Proc. Natl. Acad. Sci. USA 105:3011-6 (2008)). PD-1 and similar family members are type I transmembrane glycoproteins containing the Ig variant (V-type) domain responsible for ligand binding and the cytoplasmic tail region responsible for binding signal transduction molecules. The cytoplasmic tail region of PD-1 contains two tyrosine-based signal transduction motifs, ITIM (immune receptor tyrosine-based inibitorymotif) and ITSM (immune receptor tyrosine-based switchmotif).

PD-1 plays an important role in the immune escape mechanism of tumors. Tumor immunotherapy, that is, using the body's own immune system to fight cancer, is a breakthrough tumor therapy, but the tumor microenvironment can protect tumor cells from effective immune destruction. Therefore, how to break the tumor microenvironment has become the focus of anti-tumor research. Existing studies have identified the role of PD-1 in the tumor microenvironment: PD-L1 is expressed in many mouse and human tumors (and can be induced by IFN-γ in most PD-L1 negative tumor cell lines) and is presumed to be an important target mediating tumor immune escape (Iwai Y. et al., Proc. Natl. Acad. Sci. U.S.A. 99:12293-12297 (2002); Strome S.E. et al., Cancer Res., 63:6501-6505(2003)). Expression of PD-1 (on tumor-infiltrating lymphocytes) and/or PD-L1 on tumor cells has been found in many human primary tumors by immunohistochemical evaluation biopsies. Such tissues include lung cancer, liver cancer, ovarian cancer, cervical cancer, skin cancer, colon cancer, glioma, bladder cancer, breast cancer, kidney cancer, esophagus cancer, stomach cancer, oral squamous cell carcinoma, urethral epithelial cell carcinoma and pancreatic cancer, as well as head and neck tumors, et al. It can be seen that blocking the interaction of PD-1/PD-L1 can improve the immune activity of tumor-specific T cells and help the immune system to clear tumor cells. Therefore, PD-L1 has become a popular target for the development of tumor immunotherapy drugs.

EGFR (Epidermal Growth Factor Receptor) is a receptor of epidermal growth factor (EGF), which belongs to the ErbB receptor family. EGFR is a transmembrane glycoprotein with a molecular weight of 170KDa, which is a receptor tyrosine kinase. Under the function of related ligands such as EGF and transforming growth factor α (TGFα), EGFR is transformed from monomer to dimer and activated, thus further activating downstream signaling pathways and regulating cell proliferation. A large number of studies have shown that EGFR is overexpressed or abnormally expressed in most tumors such as glial cell carcinoma, kidney cancer, lung cancer, prostate cancer, pancreatic cancer, breast cancer and other tissues. Abnormal EGFR function is associated with tumor cell proliferation, angiogenesis, tumor invasion, metastasis, and inhibition of cell apoptosis. The abnormal function of EGFR is mainly manifested in two aspects: one is excessive abnormal expression in tumor tissues, and the other is persistent activation of EGFR mutants in tumor cells (without ligand stimulation or formation of self-circulating stimulation pathways). The expression rate of EGFR in colon cancer patients ranges from 25 to 77%. Relevant clinical data showed that the level of EGFR expression is closely related to the degree of tumor malignancy and the prognosis of tumor patients.

Erbitux (Cetuximab, IMC-C225) is a mouse and human chimeric monoclonal antibody against EGFR that binds specifically to EGFR and competitively blocks EFGR from binding to its ligand, thereby inhibiting EGFR signaling. Erbitux was approved for sale in February 2004. It is used in combination with Irinotecan for the treatment of EGFR-positive metastatic colorectal cancer that has not responded to irinotecan chemotherapy, and in combination with radiotherapy for the treatment of local early squamous cell carcinoma of the head and neck (SCCHN).

Bispecific antibodies are gradually becoming a new class of therapeutic antibodies, which can be used to treat various inflammatory diseases, cancer and other diseases.

There is an urgent need in this art to develop excellent bispecific antibodies for the treatment of diseases such as cancer.

### SUMMARY OF THE INVENTION

The present invention provides an anti-PDL1 × EGFR bispecific antibody and use thereof.

Therefore, the first purpose of the present invention is to provide an anti-PDL1 × EGFR bispecific antibody.

The second purpose of the present invention is to provide an isolated nucleotide encoding the anti-PDL1 × EGFR bispecific antibody.

The third purpose of the present invention is to provide an expression vector comprising the nucleotide.

The fourth purpose of the present invention is to provide a host cell comprising the expression vector.

The fifth purpose of the present invention is to provide a preparation method for the bispecific antibody.

The sixth purpose of the present invention is to provide a pharmaceutical composition comprising the bispecific antibody.

The seventh purpose of the present invention is to provide the use of the bispecific antibody or the pharmaceutical composition in the preparation of drugs for the treatment of cancer.

The eighth purpose of the present invention is to provide a method of the bispecific antibody or the drug composition for the treatment of cancer.

In order to achieve the above purpose, the present invention provides the following technical solutions:
The first aspect of the invention provides an anti-PDL1×EGFR bispecific antibody comprising two polypeptide chains and two light chains selected from the following group:
(a) the polypeptide chain comprises
   VL-PDL1-linker1-VH-PDL1-linker2-VH-EGFR-CH1-CH2-CH3 or
   VH-PDL1-linker1-VL-PDL1-linker2-VH-EGFR-CH1-CH2-CH3 or
   EGFR-CH1-CH2-CH3-linker2-VL-PDL1-linker1-VH-PDL1 or
   EGFR-CH1-CH2-CH3-linker2-VH-PDL1-linker1-VL-PDL1 from the N terminal to the C terminal,
   the light chain comprises VL-EGFR-CL from the N terminal to the C terminal; or
(b) the polypeptide chain comprises
   VL-EGFR-linker1-VH-EGFR-linker2-VH-PDL1-CH1-CH2-CH3 or
   VH-EGFR-linker1-VL-EGFR-linker2-VH-PDL1-CH1-CH2-CH3 or
   PDL1-CH1-CH2-CH3-linker2-VL-EGFR-linker1-VH-EGFR or
   PDL1-CH1-CH2-CH3-linker2-VH-EGFR-linker1-VL-EGFR from the N terminal to the C terminal,

   the light chain comprises VL-PDL1-CL from the N terminal to the C terminal;
   wherein, the VL-PDL1 is a light chain variable region binding to PD-L1,
   the VH-PDL1 is a heavy chain variable region binding to PD-L1,
   the VL-EGFR is a light chain variable region binding to EGFR,
   the VH-EGFR is a heavy chain variable region binding to EGFR,
   the CH1-CH2-CH3 is a heavy chain constant region, the CL is a light chain constant region,
   the linker1 and linker2 are independently flexible peptide linkers,
   the VL-PDL1 and the VH-PDL1 form an antigen-binding site that specifically binds to PD-L1,
   the VH-EGFR and the VL-EGFR form an antigen-binding site that specifically binds to EGFR.

In another preferred embodiment, the bispecific antibody has the activity of binding to both EGFR and PD-L1.

In another preferred embodiment, the flexible peptide linker comprises 6-30 amino acids and preferably 10-25 amino acids.

In another preferred embodiment, the flexible peptide linker comprises 2-6 G4S and/or G3S.

In another preferred embodiment, the Linker1 is 3-5 G4S.

In another preferred embodiment, the Linker2 is 2-4 G4S.

In another preferred embodiment, the anti-PDL1×EGFR bispecific antibody comprises:
(a) Two polypeptide chains, wherein the polypeptide chain comprising VL-PDL1-linker1-VH-PDL1-linker2-VH-EGFR-CH1-CH2-CH3 from the N terminal to the C terminal,
   wherein,
   the VL-PDL1 is a light chain variable region binding to PD-L1;
   the linker1 and linker2 are independently flexible peptide linkers;
   the VH-EGFR is a heavy chain variable region binding to EGFR,
   the CH1-CH2-CH3 is a heavy chain constant region, and
(b) Two light chains, wherein the light chain comprising VL-EGFR-CL from the N terminal to the C terminal, wherein the VL-EGFR is a light chain variable region binding to EGFR, and the CL is a light chain constant region,
wherein, the VL-PDL1 and the VH-PDL1 form an antigen-binding site that specifically binds to PD-L1, and the VH-EGFR and the VL-EGFR form an antigen-binding site that specifically binds to EGFR.

In another preferred embodiment, the anti-PDL1×EGFR bispecific antibody comprises:
(a) Two polypeptide chains, wherein the polypeptide chain comprising VL-PDL1-linker1-VH-PDL1-linker2-VH-EGFR-CH1-CH2-CH3 from the N terminal to the C terminal, wherein the VL-PDL1 is a light chain variable region binding to PD-L1, the linker1 is 4 or 5 GGGGS, the VH-PDL1 is a heavy chain variable region binding to PD-L1, the linker2 is 3 or 4 GGGGS, the VH-EGFR is a heavy chain variable region binding to EGFR, the CH1-CH2-CH3 is a heavy chain constant region, and
(b) Two light chains, wherein the light chain comprising VL-EGFR-Cl from the N terminal to the C terminal, wherein the VL-EGFR is a light chain variable region binding to EGFR, and the CL is a light chain constant region,
wherein, the VL-PDL1 and the VH-PDL1 form an antigen-binding site that specifically binds to PD-L1, and the VH-EGFR and the VL-EGFR form an antigen-binding site that specifically binds to EGFR.

According to a preferred embodiment of the present invention, the VL-PDL1 comprises light chain CDRs with amino acid sequences as shown in SEQ ID NO: 1-3, and the VH-PDL1 comprises heavy chain CDRs with amino acid sequences as shown in SEQ ID NO: 4-6, and the VH-EGFR comprises heavy chain CDRs with amino acid sequence as shown in SEQ ID NO: 7-9, and the VL-EGFR comprises light chain CDRs with amino acid sequences as shown in SEQ ID NO: 10-12.

According to a preferred embodiment of the present invention, the VL-PDL1 has an amino acid sequence as shown in SEQ ID NO: 13, and the VH-PDL1 has an amino acid sequence as shown in SEQ ID NO: 14, and the VH-EGFR has an amino acid sequence as shown in SEQ ID NO: 15, and the VL-EGFR has an amino acid sequence as shown in SEQ ID NO: 16.

According to a preferred embodiment of the present invention, the polypeptide chain has an amino acid sequence as shown in SEQ ID NO: 17 or SEQ ID NO: 18 or SEQ ID NO: 23 or SEQ ID NO: 24 or SEQ ID NO: 25, and the light chain has an amino acid sequence as shown in SEQ ID NO 19;
or the polypeptide chain has an amino acid sequence as shown in SEQ ID NO: 26 or SEQ ID NO: 27 or SEQ ID NO: 28 or SEQ ID NO: 29, and the light chain has an amino acid sequence as shown in SEQ ID NO: 22.

According to the present invention, the heavy chain constant region comprises IgG1, IgG2, IgG3 or IgG4 heavy chain constant region, and the light chain constant region comprises κ or λ light chain constant region.

The second aspect of the present invention provides an isolated nucleotide encoding the bispecific antibody.

The third aspect of the present invention provides an expression vector comprising the nucleotides described above.

The fourth aspect of the present invention provides a host cell comprising the expression vector described above.

The fifth aspect of the present invention provides a preparation method for the bispecial antibody, which comprises the following steps:
(a) The host cells described above are cultured under the expression conditions to express the bispecific antibody;
(b) Isolation and purification of the bispecific antibody described in (a).

The sixth aspect of the present invention provides a pharmaceutical composition comprising the bispecific antibody as described above and a pharmaceutically acceptable carrier.

In another preferred embodiment, the pharmaceutical composition further comprises an antitumor agent.

In another preferred embodiment, the pharmaceutical composition is in unit form.

In another preferred embodiment, the antitumor agent may exist separately in a separate package with the bispecific antibody, or the antitumor agent may be conjugated with the bispecific antibody.

In another preferred embodiment, the dosage forms of the pharmaceutical composition comprise gastrointestinal or parenteral administration dosage forms.

In another preferred embodiment, the parenteral administration dosage forms comprise intravenous injection, intravenous infusion, subcutaneous injection, topical injection, intramuscular injection, intratumoral injection, intraperitoneal injection, intracranial injection, or intracavitary injection.

The seventh aspect of the present invention provides the use of the bispecific antibody, or immune conjugate thereof, or the pharmaceutical composition described above, in the preparation of drugs for the treatment of cancer.

According to the present invention, the cancer is selected from the group consisting of colorectal cancer, non-small cell lung cancer, squamous cell cancer, head and neck cancer, kidney cancer, bladder cancer, ovarian cancer, breast cancer, melanoma, lung cancer, liver cancer, gastric cancer, lymphoma, leukemia, prostate cancer, bone marrow cancer and other neoplastic malignant diseases.

The eighth aspect of the present invention provides a method for the treatment of cancer, which comprises the step of administering the bispecific antibody, or immune conjugate thereof, or the pharmaceutical composition as described above, to a subject in need.

According to the present invention, the cancer isselected from the group consisting of colorectal cancer, non-small cell lung cancer, squamous cell cancer, head and neck cancer, kidney cancer, bladder cancer, ovarian cancer, breast cancer, melanoma, lung cancer, liver cancer, gastric cancer, lymphoma, leukemia, prostate cancer, bone marrow cancer and other neoplastic malignant diseases.

The ninth aspect of the present invention provides an immune conjugate which comprisies:
(a) the bispecific antibodies according to the first aspect of the present invention; and
(b) a coupling moiety selected from the group consisting of a detectable label, a drug, a toxin, a cytokine, a radionuclide, or an enzyme.

In another preferred embodiment, the coupling moiety is selected from: a fluorescent or luminescent label, a radioactive label, MRI (magnetic resonance imaging) or CT (electronic computer tomography) contrast agent, or an enzyme capable of producing detectable products, a radionuclide, a biological toxin, a cytokine (such as IL-2).

In another preferred embodiment, the immune conjugate includes an antibody-drug conjugate (ADC).

In another preferred embodiment, the immune conjugate is used to prepare a pharmaceutical composition for the treatment of tumors.

Beneficial effects: The present invention provides an anti-PDL1×EGFR bispecific antibody. Experimental results show that the bispecific antibody can better maintain the activity of anti-EGFR and anti-PD-L1 monoclonal antibody, and can specifically bind to two targets of PD-L1 and EGFR at the same time, and has obvious synergistic effect at the cellular level.

### DESCRIPTION OF DRAWINGS

Figure 1 shows a schematic diagram of the anti-PDL1×EGFR bispecific antibody of the present invention.
Figure 2 shows the HPLC assay chromatogram and SDS-PAGE assay results of anti-PDL1×EGFR bispecific antibody, wherein Figure 2A shows the HPLC assay chromatogram and Figure 2B shows the SDS-PAGE assay results.
Figure 3 shows the results of anti-PDL1×EGFR bispecific antibodies binding to PD-L1 and EGFR respectively detected by ELISA, wherein Figure 3A shows the results of binding to PD-L1 and Figure 3B shows the results of binding to EGFR.
Figure 4 shows the results of anti-PDL1×EGFR bispecific antibodies binding to PD-L1 and EGFR simultaneously detected by ELISA.
Figure 5 shows the results of the anti-PDL1×EGFR bispecific antibody binding to A431 and N87-PDL1 cells detected by FACS, respectively, wherein Figure 5A shows the results of binding to A431 cells, and Figure 5B shows the results of binding to N87-PDL1 cells.
Figure 6 shows the results of A431 cell proliferation activity inhibited by anti-PDL1 ×EGFR antibody.
Figure 7 shows the results of PD1/PD-L1 activity in cells blocked by anti-PDL1 ×EGFR bispecific antibody.
Figure 8 shows the fitting diagram of affinity detection between anti-PDL1 ×EGFR bispecific antibody and EGFR antigen.
Figure 9 shows the fitting diagram of affinity detection between anti-PDL1 ×EGFR bispecific antibody and PD-L1 antigen.
Figure 10 shows the synergistic effect of anti-PDL1 ×EGFR bispecific antibody at the cellular level.
Figure 11 shows the antitumor effect of anti-PDL1 ×EGFR bispecific antibody on SW48 xenograft model.
Figure 12 shows the antitumor effect of anti-PDL1 ×EGFR bispecific antibody on the B-hPD-L1 humanized mouse MC38-hPD-L1 xenograft model.
Figure 13 shows the results of A431 cell proliferation activity inhibited by anti-PDL1 ×EGFR antibody in comparable example.
Figure 14 shows the results of PD1/PD-L1 activity in cells blocked by anti-PDL1 ×EGFR bispecific antibody in comparable example, wherein Figure 14A shows the activity comparison between anti-PDL1×EGFR BsAb rev1, rev2, rev3 and the positive control anti-PD-L1 monoclonal antibody, and Figure 14B shows the the activity comparison betweenanti-PDL1×EGFR BsAb rev5, rev6, rev7, rev8 and the positive control anti-PD-L1 monoclonal antibody.

### DETAILED DESCRIPTION

Through extensive and intensive research and a large number of screening, the inventor obtains a bispecific antibody, which consists of anti-EGFR antibody and anti-PD-L1 antibody. The bispecific antibody of the present invention belongs to a homologous dimer. The bispecific antibody of the present invention not only maintains the activities of anti-EGFR antibody and anti-PDL1 antibody, but also can bind to EGFR and PD-L1 at the same time. More importantly, cellular experiments show that the bispecific antibody hasa synergistic effect on killing tumor cells, and shows significantly greater killing activity than that of the anti-EGFR antibody or the anti-PD-L1 antibody. Therefore, the bispecific antibody of the present invention can be developed as an antitumor drug with superior efficacy. The present invention has been completed on this basis.

### Term

In the present invention, the term "antibody (Ab)" and "immunoglobulin G (IgG)" is a heterotetrameric glycoprotein having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a constant region, and heavy chain constant region consists of three domains CH1, CH2, and CH3. Each light chain has a variable region (VL) at one end and a constant region at the other end, and the light chain constant region includes a CL domain. The constant region of the light chain is paired with the CH1 domain of the heavy chain constant region, and the variable region of the light chain is paired with the variable region of the heavy chain. The constant regions are not directly involved in antibody-antigen binding, but they can be used in different effect function, such as antibody-dependent cell-mediated cytotoxicity (ADCC), etc. The heavy chain constant region includes IgG1, IgG2, IgG3 and IgG4 subtypes. The light chain constant region includes κ (Kappa) or λ (Lambda). The heavy and light chains of antibody are covalently linked by disulfide bonds between the CH1 domain of the heavy chain and the CL domain of the light chain, and the two heavy chains of antibody are covalently linked by interpeptide disulfide bonds formed between hinge regions.

In the present invention, the term "bispecific antibody (or bi-antibody)" refers to an antibody molecule that can specifically bind two antigens (targets) or two epitopes simultaneously. Bispecific antibodies can be divided into structurally symmetric and asymmetrical molecules based on symmetry. Based on the number of binding sites, bispecific antibodies can be divided into bivalent, tetravalent, and multivalent molecules.

In the present invention, the term "monoclonal antibody (mAb)" refers to an antibody obtained from a population that is essentially homogeneous, that is, the individual antibodies contained in the population are identical except for a few naturally occurring mutations that may be present. Monoclonal antibodies target a single antigenic site with high specificity. Furthermore, unlike conventional polyclonal antibody preparations, which are usually mixtures of different antibodies that target different epitopes, each monoclonal antibody targets a single epitopes on the antigen. In addition to their specificity, the benefit of monoclonal antibodies is that they can be synthesized in hybridoma culture and are not contaminated with other immunoglobulins. The modifier "monoclonal" indicates the nature of an antibody, which is derived from a substantially homogeneous population of antibodies, and should not be interpreted as requiring any special method for producing antibodies.

In the present invention, the terms "Fab" and "Fc" refer to the cleavage of the antibody into two identical Fab segments and one Fc segment by papain. The Fab segment consists of VH and CH1 domains of the heavy chain and VL and CL domains of the light chain of the antibody. The Fc segment, that is fragment crystallizable, consists of the CH2 and CH3 domains of the antibody. Fc segment has no antigen-binding activity and is the site where antibodies interact with effector molecules or cells.

In the present invention, the term "scFv" refers to a single chain antibody fragment, consisting of a heavy chain variable region and a light chain variable region of an antibody usually connected by a linking short peptide (linker) of 15 to 25 amino acids.

In the present invention, the term "variable" means that antibodies are different from each other in terms of sequence in certain parts of variable regions, which is responsible for the binding and specificity of various specific antibodies to their specific antigens. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). Each of the variable regions of naturally occurring heavy and light chains comprises four FR regions, which are generally in a β-sheet configuration, connected by three CDRs forming a linking loop, and in some cases, may form a partial β-sheet structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)).

As used herein, the term "framework region" (FR) refers to amino acid sequence inserted between CDRs, i.e, those portions of the light and heavy chain variable regions of the immunoglobulins that are relatively conserved among different immunoglobulins ina single species. The light chain and heavy chain of immunoglobulin each have four FRs, which are called FR1-L, FR2-L, FR3-L, FR4-L and FR1-H, FR2-H, FR3-H and FR4-H. Accordingly, the light chain variable domain may thus be referred to as (FR1-L)-(CDR1-L)-(FR2-L)-(CDR2-L)-(FR3-L)-(CDR3-L)-(FR4-L) and the heavy chain variable domain may thus be represented as (FR1-H)-(CDR1-H)-(FR2-H)-(CDR2-H)-(FR3-H)-(CDR3-H)-(FR4-H). Preferably, the FR of the present invention is a human antibody FR or a derivative thereof, and the derivative of the human antibody FR is substantially identical to a naturally occurring human antibody FR, that is, the sequence identity reaches 85%, 90%, 95%, 96%, 97%, 98% or 99%.

Knowing the amino acid sequence of the CDR, those skilled in the art can easily determine the framework regions FR1-L, FR2-L, FR3-L, FR4-L and/or FR1-H, FR2-H, FR3-H, FR4-H.

As used herein, the term "human framework region" is a framework region that is substantially identical (about 85% or more, specifically 90%, 95%, 97%, 99% or 100%) to the framework region of a naturally occurring human antibody.

As used herein, the term "linker" refers to one or more amino acid residues inserted into the immunoglobulin domain to provide sufficient mobility for the domains of the light and heavy chains to fold into the exchange dual variable region immunoglobulin. In the present invention, the preferred linker refers to Linker1 and Linker2, wherein Linker1 links the VH and VL of a single chain antibody (scFv), and Linker2 is used to connect the scFv to the heavy chain of another antibody.

Suitable examples of linkers include a single glycine (Gly) or serine (Ser) residue. The identification and sequence of amino acid residues in a linker may vary depending on the type of secondary structural element to be implemented in the linker.

### Bispecific antibody

The bispecific antibody of the present invention is an anti-PDL1×EGFR bispecific antibody, which comprises an anti-PDL1 antibody part and an anti-EGFR antibody part.

Preferably, the sequence of the anti-PD-L1 antibody of the present invention is described in the patent application PCT/CN2020/090442, and those skilled in the art can modify or engineer the anti-PD-L1 antibody of the present invention through the technology known in the art, such as by adding, deleting and/or substituting one or several amino acid residues. Thus, the affinity or structural stability of anti-PD-L1 antibody can be further improved, and the modified or engineered results can be obtained by conventional determination methods.

In the present invention, "conservative variant of the antibody of the present invention" refers to a polypeptide formed by replacing at most 10, preferably at most 8, more preferably at most 5, and most preferably at most 3 amino acids with amino acids of similar properties as compared with the amino acid sequence of the antibody of the present invention. These conservative variant polypeptides are preferably produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

In the present invention, the terms "anti", "binding" and "specific binding" refer to the non-random binding reaction between two molecules, such as the reaction between an antibody and the antigen it targets. Typically, antibodies bind to the antigen at an equilibrium dissociation constant (KD) of less than about 10⁻⁷M, for example, less than about 10⁻⁸M, 10⁻⁹M, 10⁻¹⁰M, 10⁻¹¹M, or less. In the present invention, the term "KD" refers to the equilibrium dissociation constant of a specific antibody-antigen interaction, which is used to describe the binding affinity between the antibody and the antigen. The smaller the equilibrium dissociation constant is, the closer the antibody-antigen binding is, and the higher the affinity between antibody and antigen is. For example, the binding affinity of an antibody to an antigen is determined using Surface Plasmon Resonance (SPR) in a BIACORE apparatus or the relative affinity of an antibody to an antigen is determined using an ELISA.

In the present invention, the term "epitope" refers to a polypeptide determinant that binds specifically to an antibody. The epitope of the present invention is a region of an antigen bound by an antibody.

The bispecial antibody of the present invention may be used alone or in combination or conjugation with detectable markers (for diagnostic purposes), therapeutic agents, or any combination of these substances.

### Coding nucleic acid and expression vector

The present invention also provides a polynucleotide molecule encoding the antibody or fragment or fusion protein thereof. The polynucleotides of the present invention may be in DNA form or RNA form. DNA forms include cDNA, genomic DNA, or synthetic DNA. DNA may be single stranded or double stranded. DNA may be a coding strand or a noncoding strand.

As used herein, the term "expression vector" means a vector carrying an expression cassette for the expression of a specific target protein or other substance, such as a plasmid, a viral vector (e.g., adenovirus, retrovirus), a phage, a yeast plasmid or other vectors. Representative examples include but are not limited to: pTT5, pSECtag series, pCGS3 series, pcDNA series vectors, and other vectors used for mammalian expression systems, etc. The expression vectors include fusion DNA sequences linked to appropriate transcriptional and translational regulatory sequences.

Once the relevant sequence is obtained, it can be obtained in large quantities by recombination method. This is usually done by cloning it into a vector, then transferring it into a cell, and then isolating the relevant sequence from the proliferating host cell by conventional methods.

The present invention also relates to a vector comprising the appropriate DNA sequence as described above and an appropriate promoter or control sequence. These vectors can be used to transform appropriate host cells to enable them to express proteins.

In the present invention, the term "host cell" refers to cells suitable for expressing the expression vectors described above, which may be eukaryotic cells, such as mammalian or insect host cell culture systems for the expression of fusion proteins of the present invention, CHO (Chinese Hamster Ovary), HEK293, COS, BHK and derived cells thereof are available for use in the present invention.

### Pharmaceutical composition and use

The present invention also provides a composition. Preferably, the composition is a pharmaceutical composition containing the above-mentioned antibody or active fragment thereof or fusion protein thereof, and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to) intravenous injection, intravenous drip, subcutaneous injection, topical injection, intramuscular injection, intratumoral injection, intraperitoneal injection (such as intraperitoneal injection), intracranial injection, or intracavitary injection..

As used herein, the term "pharmaceutical composition" refers to the bispecificbispecific antibody of the present invention can be combined with a pharmaceutically acceptable carrier to form a drug preparation composition so as to exert a more stable curative effect. These preparations can ensure the conformational integrity of the core amino acid sequence of the bispecificbispecific antibody disclosed in the present invention. They also protects the multifunctional groups of proteins from degradation (including but not limited to coagulation, deamination or oxidation).

The pharmaceutical composition of the present invention contains a safe and effective amount (e.g., 0.001-99wt%, preferably 0.01-90wt%, more preferably 0.1-80wt%) of the above-mentioned bispecific antibody of the present invention (or the conjugate thereof) and a pharmaceutically acceptable carrier or excipient. Such carriers include, but are not limited to, saline, buffer, glucose, water, glycerol, ethanol, and combinations thereof. The pharmaceutical formulation should match the mode of administration. The pharmaceutical composition of the present invention may be prepared in the form of an injection, for example, by conventional methods using normal saline or aqueous solutions containing glucose and other adjuvants. The pharmaceutical composition such as an injection and solution should be manufactured under sterile conditions. The dosage of the active ingredient is a therapeutically effective amount, for example, about 10 µg/kg body weight per day to about 50 mg/kg body weight per day. In addition, the bispecificbispecific antibody can also be used together with other therapeutic agents.

When a pharmaceutical composition is used, a safe and effective amount of the bispecific antibody or the immune conjugate thereof is administered to a mammal, wherein the safe and effective amount is typically at least about 10 µg/kg body weight, and in most cases no more than about 50 mg/kg body weight, preferably about 10 µg/kg body weight to about 10 mg/kg body weight. Of course, the specific dosage should also consider factors such as the administration route and the patient's health status, which are all within the skill range of a skilled physician.

The experimental materials used in the following examples are described as follows:
pcDNA^{™} 3.4 TOPO^{®} vector: purchased from Thermo fisher, cat no. A14697;
CHO cells: purchased from Thermo fisher, cat no. A29133;
293E cells: purchased from the NRC biotechnology Research Institute;
PD-1/PD-L1 Blockade Bioassay, Propagation model: purchased from Promega, cat no. J1252;
Human gastric cancer cell line NCI-N87: purchased from the American Typical Culture Center (ATCC);
Human epidermal cancer cell A431: purchased from ATCC;
SD rats: purchased from Shanghai Lingchang Biotechnology Co., LTD.;
SW48 cells: purchased from Shanghai Institutes for Biological Sciences, Chinese Academy of Sciences;
MC38 cells: purchased from Guangzhou Genio Biotechnology Co., LTD.
B-hPD-L1 humanized mouse: purchased from Bioxetu Jiangsu Gene Biotechnology Co., LTD.

The experimental reagents used in the following examples are described below:
Anti-PD-L1 mAb: prepared according to the sequence in PCT/CN2020/090442;
Anti-EGFR mAb: prepared according to the sequence of cetuximab;
HRP labeled goat anti-human Fc antibody: purchased from sigma, cat no. A0170;
HRP labeled mouse anti-human Fab antibody: purchased from sigma, cat no. A0293;
HRP labeled anti-6×His antibody: purchased from abcam, cat no. ab178563;
Goat Anti-Human IgG-FITC: purchased from sigma, cat no. F4143;
PBS: purchased from Bioengineering (Shanghai) Co., LTD., cat no. B548117;
PBST: PBS+0.05%Tween 20;
BSA: purchased from Bioengineering (Shanghai) Co., LTD., cat no. A60332;
FBS: purchased from Gibco, cat no. 10099;
TMB: purchased from BD Company, cat no. 555214;
RPMI 1640 medium: purchased from gibco, cat no. 22400-089;
Bio-Glo Luciferase Assay System: purchased from Promega, cat no. G7940;
Cell Counting Kit-8: Tongren Institute of Chemistry (Dojindo), Japan, cat no. CK04;
DMEM culture-medium: purchased from Cellgro, cat no. 10-013-CV.

The experimental instruments used in the following embodiments are described as follows:
PCR instrument: purchased from BioRad, cat no. C1000 Touch Thermal Cycler;
HiTrap MabSelectSuRe Column: purchased from GE Company, cat no. 11-0034-95;
Beckman Coulter CytoFLEX flow cytometer: purchased from Beckman, Inc.
SpectraMax i3x Microplate Reader, purchased from Molecular Devices, Inc.
SpectraMaxM5 Microplate Reader, purchased from Molecular Devices, Inc.

The following examples and experimental examples are further explanations of the present invention and shall not be construed as limitations of the present invention. Examples do not include detailed descriptions of traditional methods, such as those used to construct vectors and plasmids, methods for inserting protein-coding genes into such vectors and plasmids or methods for introducing plasmids into host cells. Such methods are well known to people with ordinary skills in the art and has been described in many publications, including Sambrook, J., Fritsch, E. F. and Maniais, T. (1989) Molecular Cloning: A Laboratory Manual, 2nd edition, Cold spring Harbor Laboratory Press. Experimental methods not specified in the following examples are usually subjected to conventional conditions or those recommended by the manufacturer. Unless otherwise stated, percentages and parts are calculated by weight.

### Example 1 Construction of anti-PDL1×EGFR bispectic antibody

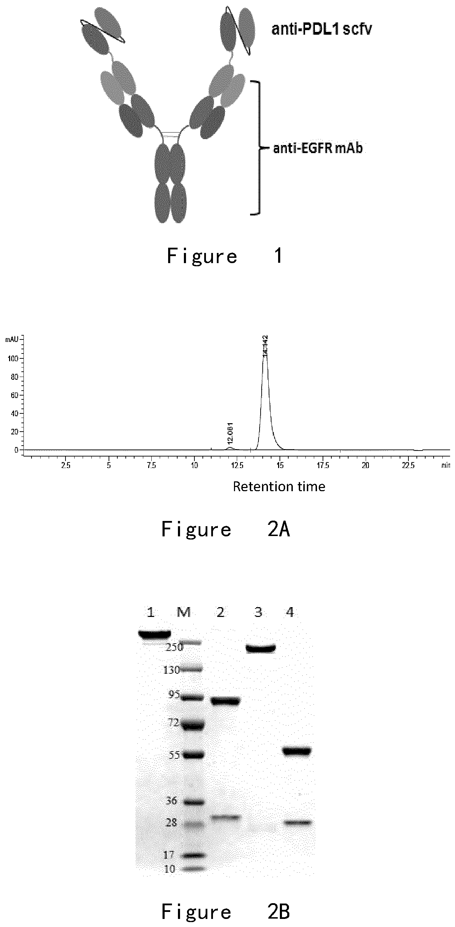

The present invention adopts the method that scFv (VL-linker1-VH, linker1 is 4 or 5 GGGGS) of human anti-PD-L1 monoclonal antibody M8 (sequence is derived from PCT/CN2020/090442) is connected to the N-terminal of the heavy chain of anti-human EGFR monoclonal antibody (prepared according to cetuximab sequence, the sequence is derived from Magdelaine-Beuzelin C, Kaas Q, Wehbi V,et al. Structure-function relationships of the variable domains of monoclonal antibodies approved for cancer treatment[J]. Critical reviews in oncology/hematology, 2007, 64(3): 210-225.) in tandem by linker2 (3 or 4 GGGGS) to construct an anti-PDL1×EGFR bispecific antibody. Wherein, the bispectic antibody of linker1 with 4 GGGGS and linker2 with 3 GGGGS is named as anti-PDL1×EGFR BsAb1. The bispectic antibody of linker1 with 5 GGGGS and linker2 with 3 GGGGS is named as anti-PDL1×EGFR BsAb2. The structures are shown in Figure 1.

The present invention also adopts several other connection modes to construct anti-PDL1×EGFR bispectic antibodies, as shown below:
anti-PDL1×EGFR BsAb rev1: The scFv of anti-human PD-L1 monoclonal antibody (VL-linker1-VH from the N terminal to the C terminal, linker1 is 4 GGGGS) is used, and the VL of scFv is connected to the C terminal of the heavy chain of anti-human EGFR monoclonal antibody in tandem by linker2 (3 GGGGS).
anti-PDL1×EGFR BsAb rev2: The scFv of anti-human PD-L1 monoclonal antibody (VH-linker1-VL from the N terminal to the C terminal, linker1 is 4 GGGGS) is used, and the VH of scFv is connected to the C terminal of the heavy chain of anti-human EGFR monoclonal antibody in tandem by linker2 (3 GGGGS).
anti-PDL1×EGFR BsAb rev3: The scFv of anti-human PD-L1 monoclonal antibody (VH-linker1-VL from the N terminal to the C terminal, linker1 is 4 GGGGS) is used, and the VL of scFv is connected to the N-terminal of the heavy chain of anti-human EGFR monoclonal antibody in tandem by linker2 (3 GGGGS).
anti-PDL1×EGFR BsAb rev5: The scFv of anti-human EGFR monoclonal antibody (VL-Linker1-VH from the N terminal to the C terminal, linker1 is 4 GGGGS) is used, and the VL of scFv is connected to the C- terminal of the heavy chain of anti-human PD-L1 monoclonal antibody.
anti-PDL1×EGFR BsAb rev6: The scFv of anti-human EGFR monoclonal antibody (Vh-Linker1-VL from the N terminal to the C terminal, linker1 was 4 GGGGS) was used, and the VH of scFv was connected by linker2 (3 GGGGS) in tandem to the C- terminal of the heavy chain of anti-human PD-L1 monoclonal antibody in tandem by linker2 (3 GGGGS).
anti-PDL1×EGFR BsAb rev7: The scFv of anti-human EGFR monoclonal antibody (VH-linker1-VL from the N terminal to the C terminal, linker1 is 4 GGGGS) is used, and the VL of scFv is connected the N-terminal of the heavy chain of anti-human PD-L1 monoclonal antibody in tandem by linker2 (3 GGGGS).
anti-PDL1×EGFR BsAb rev8: The scFv of anti-human EGFR monoclonal antibody (VL-linker1-VH from the N terminal to the C terminal, linker1 is 4 GGGGS) is used, and the VH of scFv is connected to the N-terminal of the heavy chain of anti-human PD-L1 monoclonal antibody in tandem by linker2 (3 GGGGS).

The heavy chain and light chain expression vectors of the bispecific antibody and corresponding monoclonal antibody thereof were obtained by gene synthesis and conventional molecular cloning methods. The corresponding amino acid sequences are shown in Table 1, in which CDR is encoded according to the Kabat rule.

**Table 1 Antibody Sequence information of the present invention**

| SEQ ID NO: | Sequence name | Sequence |
|---|---|---|
| 1 | Amino acid sequence of light chain complementary determinant L-CDR1 of anti-PD-L1 monoclonal antibody | RASQSIGTTIH |
| 2 | Amino acid sequence of light chain complementary determinant L-CDR2 of anti-PD-L1 monoclonal antibody | YASQSFS |
| 3 | Amino acid sequence of light chain complementary determinant L-CDR3 of anti-PD-L1 monoclonal antibody | QQSNSWPLT |
| 4 | Amino acid sequence of heavy chain complementary determinant H-CDR1 of anti-PD-L1 monoclonal antibody | SYGVH |
| 5 | Amino acid sequence of heavy chain complementary determinant H-CDR2 of anti-PD-L1 monoclonal antibody | LIWSGGGTDYNPSLKS |
| 6 | Amino acid sequence of heavy chain complementary determinant H-CDR3 of anti-PD-L1 monoclonal antibody | QLGLRAMDY |
| 7 | Amino acid sequence of the heavy chain complementary determinant H-CDR1 of anti-EGFR monoclonal antibody | GFSLTNYGVH |
| 8 | Amino acid sequence of the heavy chain complementary determinant H-CDR2 of anti-EGFR monoclonal antibody | VIWSGGNTDYNTPFTS |
| 9 | Amino acid sequence of the heavy chain complementary determinant H-CDR3 of anti-EGFR monoclonal antibody | ALTYYDYEFAY |
| 10 | Amino acid sequence of the light chain complementary determinant L-CDR1 of anti-EGFR monoclonal antibody | RASQSIGTNIH |
| 11 | Amino acid sequence of the light chain complementary determinant L-CDR2 of anti-EGFR monoclonal antibody | YASESIS |
| 12 | Amino acid sequence of the light chain complementary determinant L-CDR3 of anti-EGFR monoclonal antibody | QQNNNWPTT |
| 13 | Amino acid sequence of the light chain variable region of anti-PD-L1 monoclonal antibody | EIVLTQSPDFLSVTPKEKVTITCRASQSIGTTIHWYQQ KPDQSPKLLIKYASQSFSGVPSRFSGSGSGTDFTLTIN SVEAEDAATYYCQQSNSWPLTFGAGTKLEIK |
| 14 | Amino acid sequence of the heavy chain variable region of anti-PD-L1 monoclonal antibody | QVQLQQSGGGLVKPSQSLSLTCTVSGFSLTSYGVHW VRQPPGKGLEWIGLIWSGGGTDYNPSLKSRLTISRDT |
| | | SKNQVSFKISSLTAADTAVYYCARQLGLRAMDYWG QGTSVTVSS |
| 15 | Amino acid sequence of the heavy chain variable region of anti-EGFR monoclonal antibody | QVQLKQSGPGLVQPSQSLSITCTVSGFSLTNYGVHW VRQSPGKGLEWLGVIWSGGNTDYNTPFTSRLSINKD NSKSQVFFKMNSLQSNDTAIYYCARALTYYDYEFA YWGQGTLVTVSA |
| 16 | Amino acid sequence of light chain variable region of anti-EGFR monoclonal antibody | DILLTQSPVILSVSPGERVSFSCRASQSIGTNIHWYQQ RTNGSPRLLIKYASESISGIPSRFSGSGSGTDFTLSINS VESEDIADYYCQQNNNWPTTFGAGTKLELK |
| 17 | Amino acid sequence of anti-PDL1×EGFR BsAb1 polypeptide chain | EIVLTQSPDFLSVTPKEKVTITCRASQSIGTTIHWYQQ KPDQSPKLLIKYASQSFSGVPSRFSGSGSGTDFTLTIN SVEAEDAATYYCQQSNSWPLTFGAGTKLEIKGGGG SGGGGSGGGGSGGGGSQVQLQQSGGGLVKPSQSLS LTCTVSGFSLTSYGVHWVRQPPGKGLEWIGLIWSGG GTDYNPSLKSRLTISRDTSKNQVSFKISSLTAADTAV YYCARQLGLRAMDYWGQGTSVTVSSGGGGSGGGG SGGGGSQVQLKQSGPGLVQPSQSLSITCTVSGFSLTN YGVHWVRQSPGKGLEWLGVIWSGGNTDYNTPFTSR LSINKDNSKSQVFFKMNSLQSNDTAIYYCARALTYY DYEFAYWGQGTLVTVSAASTKGPSVFPLAPSSKSTS GGTAALGCLVKDYFPEPVTVSWNSGALTSGVHTFP AVLQSSGLYSLSSVVTVPSSSLGTQTYICNVNHKPSN TKVDKKVEPKSCDKTHTCPPCPAPELLGGPSVFLFPP KPKDTLMISRTPEVTCVVVDVSHEDPEVKFNWYVD GVEVHNAKTKPREEQYNSTYRVVSVLTVLHQDWL NGKEYKCKVSNKALPAPIEKTISKAKGQPREPQVYT LPPSREEMTKNQVSLTCLVKGFYPSDIAVEWESNGQ PENNYKTTPPVLDSDGSFFLYSKLTVDKSRWQQGN VFSCSVMHEALHNHYTQKSLSLSPGK |
| 18 | Amino acid sequence of anti-PDL1×EGFR BsAb2 polypeptide chain | EIVLTQSPDFLSVTPKEKVTITCRASQSIGTTIHWYQQ KPDQSPKLLIKYASQSFSGVPSRFSGSGSGTDFTLTIN SVEAEDAATYYCQQSNSWPLTFGAGTKLEIKGGGG SGGGGSGGGGSGGGGSGGGGSQVQLQQSGGGLVK PSQSLSLTCTVSGFSLTSYGVHWVRQPPGKGLEWIG LIWSGGGTDYNPSLKSRLTISRDTSKNQVSFKISSLTA ADTAVYYCARQLGLRAMDYWGQGTSVTVSSGGGG SGGGGSGGGGSQVQLKQSGPGLVQPSQSLSITCTVS GFSLTNYGVHWVRQSPGKGLEWLGVIWSGGNTDY NTPFTSRLSINKDNSKSQVFFKMNSLQSNDTAIYYCA RALTYYDYEFAYWGQGTLVTVSAASTKGPSVFPLA PSSKSTSGGTAALGCLVKDYFPEPVTVSWNSGALTS GVHTFPAVLQSSGLYSLSSVVTVPSSSLGTQTYICNV NHKPSNTKVDKKVEPKSCDKTHTCPPCPAPELLGGP SVFLFPPKPKDTLMISRTPEVTCVVVDVSHEDPEVKF NWYVDGVEVHNAKTKPREEQYNSTYRVVSVLTVL HQDWLNGKEYKCKVSNKALPAPIEKTISKAKGQPRE PQVYTLPPSREEMTKNQVSLTCLVKGFYPSDIAVEW ESNGQPENNYKTTPPVLDSDGSFFLYSKLTVDKSRW QQGNVFSCSVMHEALHNHYTQKSLSLSPGK |
| 19 | The light chain amino acid sequence of anti-EGFR monoclonal antibody | DILLTQSPVILSVSPGERVSFSCRASQSIGTNIHWYQQ RTNGSPRLLIKYASESISGIPSRFSGSGSGTDFTLSINS VESEDIADYYCQQNNNWPTTFGAGTKLELKRTVAA PSVFIFPPSDEQLKSGTASVVCLLNNFYPREAKVQW KVDNALQSGNSQESVTEQDSKDSTYSLSSTLTLSKA DYEKHKVYACEVTHQGLSSPVTKSFNRGEC |
| 20 | The heavy chain amino acid sequence of anti-EGFR monoclonal antibody | QVQLKQSGPGLVQPSQSLSITCTVSGFSLTNYGVHW VRQSPGKGLEWLGVIWSGGNTDYNTPFTSRLSINKD NSKSQVFFKMNSLQSNDTAIYYCARALTYYDYEFA YWGQGTLVTVSAASTKGPSVFPLAPSSKSTSGGTAA LGCLVKDYFPEPVTVSWNSGALTSGVHTFPAVLQSS GLYSLSSVVTVPSSSLGTQTYICNVNHKPSNTKVDK KVEPKSCDKTHTCPPCPAPELLGGPSVFLFPPKPKDT LMISRTPEVTCVVVDVSHEDPEVKFNWYVDGVEVH NAKTKPREEQYNSTYRVVSVLTVLHQDWLNGKEY KCKVSNKALPAPIEKTISKAKGQPREPQVYTLPPSRE EMTKNQVSLTCLVKGFYPSDIAVEWESNGQPENNY KTTPPVLDSDGSFFLYSKLTVDKSRWQQGNVFSCSV MHEALHNHYTQKSLSLSPGK |
| 21 | The heavy chain amino acid sequence of anti-PD-L1 monoclonal antibody | QVQLQQSGGGLVKPSQSLSLTCTVSGFSLTSYGVHW VRQPPGKGLEWIGLIWSGGGTDYNPSLKSRLTISRDT SKNQVSFKISSLTAADTAVYYCARQLGLRAMDYWG QGTSVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKKVEP KSCDKTHTCPPCPAPELLGGPSVFLFPPKPKDTLMIS RTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSREEMTK NQVSLTCLVKGFYPSDIAVEWESNGQPENNYKTTPP VLDSDGSFFLYSKLTVDKSRWQQGNVFSCSVMHEA LHNHYTQKSLSLSPGK |
| 22 | The light chain amino acid sequence of anti-PD-L1 monoclonal antibody | EIVLTQSPDFLSVTPKEKVTITCRASQSIGTTIHWYQQ KPDQSPKLLIKYASQSFSGVPSRFSGSGSGTDFTLTIN SVEAEDAATYYCQQSNSWPLTFGAGTKLEIKRTVA APSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC |
| 23 | Amino acid sequence of anti-PDL1×EGFR BsAb rev1 polypeptide chain | QVQLKQSGPGLVQPSQSLSITCTVSGFSLTNYGVHW VRQSPGKGLEWLGVIWSGGNTDYNTPFTSRLSINKD NSKSQVFFKMNSLQSNDTAIYYCARALTYYDYEFA YWGQGTLVTVSAASTKGPSVFPLAPSSKSTSGGTAA LGCLVKDYFPEPVTVSWNSGALTSGVHTFPAVLQSS GLYSLSSVVTVPSSSLGTQTYICNVNHKPSNTKVDK KVEPKSCDKTHTCPPCPAPELLGGPSVFLFPPKPKDT LMISRTPEVTCVVVDVSHEDPEVKFNWYVDGVEVH NAKTKPREEQYNSTYRVVSVLTVLHQDWLNGKEY KCKVSNKALPAPIEKTISKAKGQPREPQVYTLPPSRE EMTKNQVSLTCLVKGFYPSDIAVEWESNGQPENNY KTTPPVLDSDGSFFLYSKLTVDKSRWQQGNVFSCSV MHEALHNHYTQKSLSLSPGKGGGGSGGGGSGGGGS EIVLTQSPDFLSVTPKEKVTITCRASQSIGTTIHWYQQ KPDQSPKLLIKYASQSFSGVPSRFSGSGSGTDFTLTIN SVEAEDAATYYCQQSNSWPLTFGAGTKLEIKGGGG SGGGGSGGGGSGGGGSQVQLQQSGGGLVKPSQSLS LTCTVSGFSLTSYGVHWVRQPPGKGLEWIGLIWSGG GTDYNPSLKSRLTISRDTSKNQVSFKISSLTAADTAV YYCARQLGLRAMDYWGQGTSVTVSS |
| 24 | Amino acid sequence of anti-PDL1 ×EGFR BsAb rev2 polypeptide chain | QVQLKQSGPGLVQPSQSLSITCTVSGFSLTNYGVHW VRQSPGKGLEWLGVIWSGGNTDYNTPFTSRLSINKD NSKSQVFFKMNSLQSNDTAIYYCARALTYYDYEFA YWGQGTLVTVSAASTKGPSVFPLAPSSKSTSGGTAA |
| | | LGCLVKDYFPEPVTVSWNSGALTSGVHTFPAVLQSS GLYSLSSVVTVPSSSLGTQTYICNVNHKPSNTKVDK KVEPKSCDKTHTCPPCPAPELLGGPSVFLFPPKPKDT LMISRTPEVTCVVVDVSHEDPEVKFNWYVDGVEVH NAKTKPREEQYNSTYRVVSVLTVLHQDWLNGKEY KCKVSNKALPAPIEKTISKAKGQPREPQVYTLPPSRE EMTKNQVSLTCLVKGFYPSDIAVEWESNGQPENNY KTTPPVLDSDGSFFLYSKLTVDKSRWQQGNVFSCSV MHEALHNHYTQKSLSLSPGKGGGGSGGGGSGGGGS QVQLQQSGGGLVKPSQSLSLTCTVSGFSLTSYGVHW VRQPPGKGLEWIGLIWSGGGTDYNPSLKSRLTISRDT SKNQVSFKISSLTAADTAVYYCARQLGLRAMDYWG QGTSVTVSSGGGGSGGGGSGGGGSGGGGSEIVLTQS PDFLSVTPKEKVTITCRASQSIGTTIHWYQQKPDQSP KLLIKYASQSFSGVPSRFSGSGSGTDFTLTINSVEAED AATYYCQQSNSWPLTFGAGTKLEIK |
| 25 | Amino acid sequence of anti-PDL1 ×EGFR BsAb rev3 polypeptide chain | QVQLQQSGGGLVKPSQSLSLTCTVSGFSLTSYGVHW VRQPPGKGLEWIGLIWSGGGTDYNPSLKSRLTISRDT SKNQVSFKISSLTAADTAVYYCARQLGLRAMDYWG QGTSVTVSSGGGGSGGGGSGGGGSGGGGSEIVLTQS PDFLSVTPKEKVTITCRASQSIGTTIHWYQQKPDQSP KLLIKYASQSFSGVPSRFSGSGSGTDFTLTINSVEAED AATYYCQQSNSWPLTFGAGTKLEIKGGGGSGGGGS GGGGSQVQLKQSGPGLVQPSQSLSITCTVSGFSLTNY GVHWVRQSPGKGLEWLGVIWSGGNTDYNTPFTSRL SINKDNSKSQVFFKMNSLQSNDTAIYYCARALTYYD YEFAYWGQGTLVTVSAASTKGPSVFPLAPSSKSTSG GTAALGCLVKDYFPEPVTVSWNSGALTSGVHTFPA VLQSSGLYSLSSVVTVPSSSLGTQTYICNVNHKPSNT KVDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSREEMTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 26 | Amino acid sequence of anti-PDL1 ×EGFR BsAb rev5 polypeptide chain | QVQLQQSGGGLVKPSQSLSLTCTVSGFSLTSYGVHW VRQPPGKGLEWIGLIWSGGGTDYNPSLKSRLTISRDT SKNQVSFKISSLTAADTAVYYCARQLGLRAMDYWG QGTSVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKKVEP KSCDKTHTCPPCPAPELLGGPSVFLFPPKPKDTLMIS RTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSREEMTK NQVSLTCLVKGFYPSDIAVEWESNGQPENNYKTTPP VLDSDGSFFLYSKLTVDKSRWQQGNVFSCSVMHEA LHNHYTQKSLSLSPGKGGGGSGGGGSGGGGSDILLT QSPVILSVSPGERVSFSCRASQSIGTNIHWYQQRTNG SPRLLIKYASESISGIPSRFSGSGSGTDFTLSINSVESE DIADYYCQQNNNWPTTFGAGTKLELKGGGGSGGG GSGGGGSGGGGSQVQLKQSGPGLVQPSQSLSITCTV SGFSLTNYGVHWVRQSPGKGLEWLGVIWSGGNTDY NTPFTSRLSINKDNSKSQVFFKMNSLQSNDTAIYYCA RALTYYDYEFAYWGQGTLVTVSA |
| 27 | Amino acid sequence of anti-PDL1 ×EGFR BsAb rev6 polypeptide chain | QVQLQQSGGGLVKPSQSLSLTCTVSGFSLTSYGVHW VRQPPGKGLEWIGLIWSGGGTDYNPSLKSRLTISRDT SKNQVSFKISSLTAADTAVYYCARQLGLRAMDYWG QGTSVTVSSASTKGPSVFPLAPSSKSTSGGTAALGCL VKDYFPEPVTVSWNSGALTSGVHTFPAVLQSSGLYS LSSVVTVPSSSLGTQTYICNVNHKPSNTKVDKKVEP KSCDKTHTCPPCPAPELLGGPSVFLFPPKPKDTLMIS RTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSREEMTK NQVSLTCLVKGFYPSDIAVEWESNGQPENNYKTTPP VLDSDGSFFLYSKLTVDKSRWQQGNVFSCSVMHEA LHNHYTQKSLSLSPGKGGGGSGGGGSGGGGSQVQL KQSGPGLVQPSQSLSITCTVSGFSLTNYGVHWVRQS PGKGLEWLGVIWSGGNTDYNTPFTSRLSINKDNSKS QVFFKMNSLQSNDTAIYYCARALTYYDYEFAYWGQ GTLVTVSAGGGGSGGGGSGGGGSGGGGSDILLTQSP VILSVSPGERVSFSCRASQSIGTNIHWYQQRTNGSPR LLIKYASESISGIPSRFSGSGSGTDFTLSINSVESEDIAD YYCQQNNNWPTTFGAGTKLELK |
| 28 | Amino acid sequence of anti-PDL1 ×EGFR BsAb rev7 polypeptide chain | QVQLKQSGPGLVQPSQSLSITCTVSGFSLTNYGVHW VRQSPGKGLEWLGVIWSGGNTDYNTPFTSRLSINKD NSKSQVFFKMNSLQSNDTAIYYCARALTYYDYEFA YWGQGTLVTVSAGGGGSGGGGSGGGGSGGGGSDIL LTQSPVILSVSPGERVSFSCRASQSIGTNIHWYQQRT NGSPRLLIKYASESISGIPSRFSGSGSGTDFTLSINSVE SEDIADYYCQQNNNWPTTFGAGTKLELKGGGGSGG GGSGGGGSQVQLQQSGGGLVKPSQSLSLTCTVSGFS LTSYGVHWVRQPPGKGLEWIGLIWSGGGTDYNPSL KSRLTISRDTSKNQVSFKISSLTAADTAVYYCARQLG LRAMDYWGQGTSVTVSSASTKGPSVFPLAPSSKSTS GGTAALGCLVKDYFPEPVTVSWNSGALTSGVHTFP AVLQSSGLYSLSSVVTVPSSSLGTQTYICNVNHKPSN TKVDKKVEPKSCDKTHTCPPCPAPELLGGPSVFLFPP KPKDTLMISRTPEVTCVVVDVSHEDPEVKFNWYVD GVEVHNAKTKPREEQYNSTYRVVSVLTVLHQDWL NGKEYKCKVSNKALPAPIEKTISKAKGQPREPQVYT LPPSREEMTKNQVSLTCLVKGFYPSDIAVEWESNGQ PENNYKTTPPVLDSDGSFFLYSKLTVDKSRWQQGN VFSCSVMHEALHNHYTQKSLSLSPGK |
| 29 | Amino acid sequence of anti-PDL1 ×EGFR BsAb rev8 polypeptide chain | DILLTQSPVILSVSPGERVSFSCRASQSIGTNIHWYQQ RTNGSPRLLIKYASESISGIPSRFSGSGSGTDFTLSINS VESEDIADYYCQQNNNWPTTFGAGTKLELKGGGGS GGGGSGGGGSGGGGSQVQLKQSGPGLVQPSQSLSIT CTVSGFSLTNYGVHWVRQSPGKGLEWLGVIWSGGN TDYNTPFTSRLSINKDNSKSQVFFKMNSLQSNDTAIY YCARALTYYDYEFAYWGQGTLVTVSAGGGGSGGG GSGGGGSQVQLQQSGGGLVKPSQSLSLTCTVSGFSL TSYGVHWVRQPPGKGLEWIGLIWSGGGTDYNPSLK SRLTISRDTSKNQVSFKISSLTAADTAVYYCARQLGL RAMDYWGQGTSVTVSSASTKGPSVFPLAPSSKSTSG GTAALGCLVKDYFPEPVTVSWNSGALTSGVHTFPA VLQSSGLYSLSSVVTVPSSSLGTQTYICNVNHKPSNT KVDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL |
| | | PPSREEMTKNQVSLTCLVKGFYPSDIAVEWESNGQP ENNYKTTPPVLDSDGSFFLYSKLTVDKSRWQQGNV FSCSVMHEALHNHYTQKSLSLSPGK |

### Example 2 Expression and purification of the anti-PDL1×EGFR bispectic antibody

The heavy chain and light chain DNA fragments of anti-PDL1×EGFR bispecific antibody were subcloned into pcDNA3 .4 vector respectively, and the recombinant plasmids were extracted and co-transfected into CHO cells and/or 293E cells. After 5-7 days of cell culture, the culture medium was treated through high-speed centrifugation and vacuum filtration via microporous membrane, then loadedto HiTrap MabSelectSuRe column, eluted with elution solution containing 100mM citric acid, pH3.5, and dialyzed to PBS of pH7.4.

The purified protein was detected by HPLC. HPLC-SEC chromatograms of the anti-PDL1 ×EGFR bispecific c antibodies are shown in Figure 2A. The purity of anti-PDL1×EGFR BsAb1 and anti-PDL1×EGFR BsAb2 is over 92%, and the purity of anti-PDL1 ×EGFR BsAb2 is slightly higher than that of anti-PDL1×EGFR BsAb1.

SDS-PAGE assay results are shown in Figure 2B. Lane 1 and 2 are non-reducing and reduced SDS-PAGE of anti-PDL1×EGFR bispecific antibody, lane 3 and 4 are non-reducing and reduced SDS-PAGE of anti-EGFR monoclonal antibody. The theoretical molecular weight of the bispecific antibody is 198KD.

### Example 3 The affinity of anti-PDL1×EGFR bispectic antibodies to antigen detected by Enzyme-linked immunosorbent assay (ELISA)

### 3.1 Affinity detection to PD-L1 antigen

In order to detect the affinity of anti-PDL1×EGFR bispecific antibody to PD-L1 antigen, PDL1-ECD-His protein (According to the sequence provided by NCBI (NCBI accession number: NP_054862.1), the PD-L1 extracellular domain gene was synthesized, and the signal peptide sequence was added to the N-terminal and 6×His tag was added to the C-terminal. Then it was constructed into the expression vector by EcoRI and HindIII digestion sites, and transfected into HEK-293E cells for expression and purification) was diluted to 1000ng/ml by PBS buffer of pH7.4, and then added into the ELISA plate with 100µl/welland incubated at 4°C overnight. The plate was washed twice with PBST the next day. PBST+1%BSA was added to each well for blocking at 37°C for 1h. The plate was washed twice with PBST. Then the antibody to be detected was added with PBS+1%BSA gradient dilution. Anti-PD-L1 monoclonal antibody was used as the positive control. The initial concentration was 100nM and 12 gradients were diluted three times step by step. The plate was incubated at 37 °C for 1h; and the plate was washed by PBST twice, then the secondary antibody, goat anti-human Fc antibody labeled by HRP was added, and the plate was incubated at 37 °C for 40min. The plate was washed by PBST three times and patted dry. 100µl TMB was added to each well, and was placed at room temperature (20±5 °C) away from light for 5 minutes. 50µl 2M H₂SO4 terminating solution was added to each well to terminate the substrate reaction. OD values were read at 450nm with Microplate Reader. Data were analyzed by GraphPad Prism, and EC₅₀ was plotted and calculated.

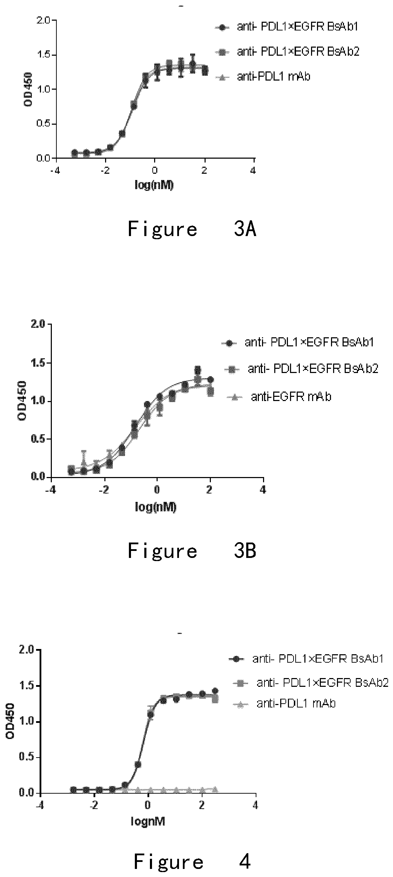

The experimental results are shown in Figure 3A. The EC₅₀ of anti-PD-L1 monoclonal antibody, anti-PDL1 ×EGFR BsAb1 and anti-PDL1 ×EGFRBsAb2 are all 0.11nM, showing similar affinity.

### 3.2 Affinity detection to EGFR antigen

In order to detect the affinity of anti-PDL1 ×EGFR bispecific antibody to EGFR antigen, EGFR-ECD-HFC protein (According to the sequence provided by UniProt (accession number P00533), extracellular gene was synthesized, and the signal peptide sequence was added to the the N-terminal and hFc tag was added to the C-terminal. Then is was constructed into the expression vector by EcoRI and HindIII digestion sites, and transfected into HEK-293E cells for expression and purification) was diluted to 1000ng/ml with PBS buffer of pH7.4, and then added into the ELISA plate with 100µl/well and incubated at 4°C overnight. The plate was washed twice with PBST the next day. PBST+1%BSA was added to each well for blocking at 37 °C for 1h. The plate was washed twice with PBST. Then the antibody to be detected was added with PBS+1%BSA gradient dilution. Anti-EGFR monoclonal antibody was used as the positive control. The initial concentration was 100nM and 12 gradients were diluted three times step by step. The plate was incubated at 37°C for 1h; and the plate was washed by PBST twice, then the secondary antibody, goat anti-human Fc antibody labeled by HRP was added, and the plate was incubated at 37 °C for 40min. The plate was washed by PBST three times and patted dry. 100µl TMB was added to each well, and was placed at room temperature (20±5 °C) away from light for 5 minutes. 50µl 2M H₂SO4 terminating solution was added to each well to terminate the substrate reaction. OD values were read at 450nm with Microplate Reader. Data were analyzed by GraphPad Prism, and EC50 was plotted and calculated.

The experimental results are shown in Figure 3B. The EC₅₀ of anti-EGFR monoclonal antibody is 0.15nM, EC₅₀ of anti-PDL1×EGFR BsAb1 is 0.15nM, and EC₅₀ of anti-PDL1×EGFR BsAb2 is 0.21nM, showing similar affinity.

### Example 4 The ability of the anti-PDL1×EGFR bispecific antibody to bind two antigens simultaneously detected by bispecific ELISA

In order to detect the ability of anti-PDL1×EGFR bispecific antibody to bind simultaneously to the EGFR antigen and PD-L1 antigen, PDL1-ECD-hFc protein (replacing the C-terminal of PDL1-ECD-His protein with an hFc tag) was diluted to 1µg/ml with PBS buffer of pH7.4. Then it was added at 100µl/well into ELISA plate and incubated at 4 °C overnight. The plate was washed twice with PBST the next day. PBST+1%BSA was added to each well for blocking at 37°C for 1h. The plate was washed twice with PBST, then the antibody to be detected was added with PBS+1%BSA gradient dilution. The initial concentration was 300nM and 12 gradients were diluted three times step by step. The plate was incubated at 37°C for 1h; and the plate was washed by PBST twice, then the secondary antibody, goat anti-human Fc antibody labeled by HRP was added, and the plate was incubated at 37 °C for 40min. The plate was washed by PBST three times and patted dry. 100µl TMB was added to each well, and was placed at room temperature (20±5 °C) away from light for 5 minutes. 50µl 2M H₂SO₄ terminating solution was added to each well to terminate the substrate reaction. OD values were read at 450nm with Microplate Reader. Data were analyzed by GraphPad Prism, and EC₅₀ was plotted and calculated.

The experimental results are shown in Figure 4. The EC₅₀ of anti-PDL1 ×EGFR BsAb1 and anti-PDL1×EGFR BsAb2 are 0.66nM and 0.63nM, respectively, while monoclonal antibody does not have the ability to bind these two antigens simultaneously.

### Example 5 The binding affinity of the anti-PDL1×EGFR bispecific antibody to target cells detected by FACS

In this study, human epidermal cancer cell A431 with high expression of EGFR on the cell surface was used as the target cells. The cells were washed three times with PBS containing 0.5% BSA, and centrifuged 300g each time for 5 minutes, and the supernatant was discarded. Cells were suspended with PBS containing 0.5% BSA to the cell density of 1×10⁶ cells/mL, and added to 96-well plates at 100µL/well. Anti-PDL1×EGFR bispecific antibody and positive control anti-EGFR monoclonal antibody were diluted to 600nM and 11 gradients were diluted step by step, then antibodies were added into 96-well plate at 100µL/well and mixed evenly with A431 cells. The cells were incubated at 4 °C for 1h, and the cells were washed twice with PBS to remove unbound antibodies to be detected. The goat anti-human IgG-FITC was added at 100µL/well and was incubated at 4 °C for 30 min. After centrifugation at 300g for 5 min, cells were washed twice with PBS to remove unbound secondary antibodies. Finally, the cells were suspended in 200µl PBS, and Beckman Coulter CytoFLEX flow cytometry was used to determine the binding affinity of the bispecific antibody to the cells. The data were fitted and analyzed by GraphPad Prism7 software.

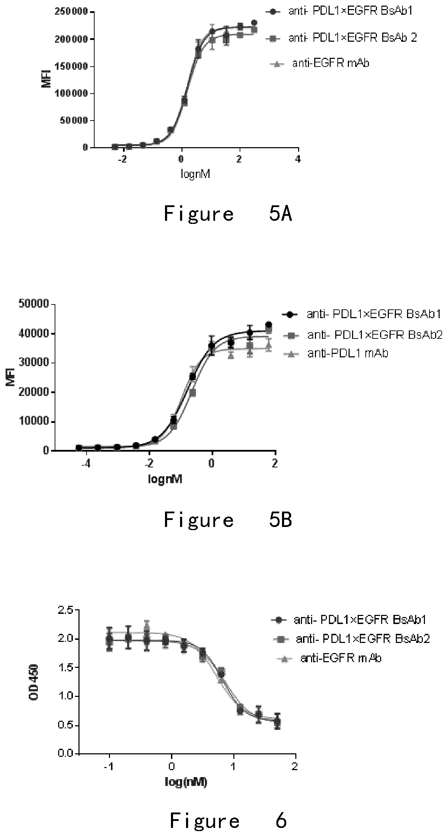

The experimental results are shown in Figure 5A. The EC₅₀ of anti-EGFR monoclonal antibody, anti-PDL1 × EGFR BsAb 1 and anti-PDL1 × EGFR BsAb2 are all 1. 56nM, showing similar affinity.

N87-PDL1 is a stable cell line constructed by PD-L1 transfected into NCI-N87 using lentivirus transfection method in our laboratory. N87-PDL1 cells at logarithmic growth stage were digested with trypsin, then washed three times with PBS containing 0.5% BSA, and centrifuged 300g for 5 minutes each time, and the supernatant was discarded. Cells were suspended with PBS containing 0.5% BSA to the cell density of 1× 10⁶ cells/mL, and added to 96-well plates at 100µL/well. The anti-PDL1×EGFR bispecific antibody and the positive control anti-PD-L1 monoclonal antibody were diluted to 120nM and 11 gradients were diluted step by step, then antibodies were added into 96-well plate at 100µL/well and mixed evenly with N87-PDL1 cells. The other methods are the same as above.

The experimental results are shown in Figure 5B. The EC₅₀ of anti-PD-L1 monoclonal antibody is 0.11nM, EC₅₀ of anti-PDL1×EGFR BsAb1 is 0.16nM, and EC₅₀ of anti-PDL1×EGFR BsAb2 is 0.22nM, showing similar affinity.

### Example 6 Inhibition of proliferation of A431 cells by anti-PDL1×EGFR bispecific antibody

EGFR is highly expressed on the surface of human epidermal cancer cell A431, and anti-EGFR antibody can effectively inhibit the proliferation of A431 cells *in vitro.* A431 cells at logarithmic growth stage were diluted to 5×10⁴/mL with RPMI 1640 medium containing 1%FBS, and inoculated into 96-well culture plates at 100µL/well. After 24h adherent growth, different concentrations of antibodies to be tested were added, which were diluted by RPMI 1640 medium containing 1% FBS. The initial working concentration of the antibody to be tested was 50nM, and 10 different concentrations were diluted three times step by step, and each concentration was set with two replicates. The cells were incubated at 37°C and 5%CO₂ for 72h, then CCK-8 chromogenic solution was added at 20µl/well and cells were incubated at room temperature for 1.5h. Readings were taken on the SpectraMaxM5 spectrometer with the detection wavelength at 450nm and the reference wavelength at 650nm. Data were analyzed by GraphPad Prism, and IC₅₀ was plotted and calculated.

The experimental results are shown in Figure 6. The IC₅₀ of anti-PDL1 ×EGFR BsAb1, anti-PDL1×EGFR BsAb2 and anti-EGFR mAb inhibiting A431 proliferation is 6.63nM, 7.17nM and 5.23nM, respectively, showing similar affinity.

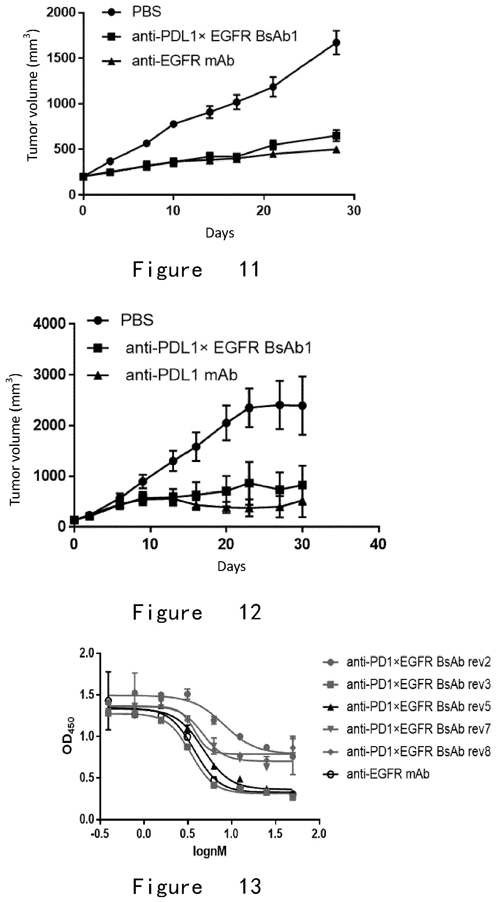

The results of *in vitro* proliferation inhibition of A431 cells by bispecific antibodies includinganti-PDL1×EGFR BsAb rev1, rev2, rev3, rev5, rev6, rev7 and rev8 are shown in Figure 13. The activity of anti-PDL1×EGFR BsAb rev2, rev7 and rev8 is significantly worse than that of the positive control, and anti-PDL1×EGFR rev5 is slightly weaker than that of the positive control. The activity of anti-PDL1×EGFR rev3 is not significantly different from that of positive control anti-EGFR monoclonal antibody.

In addition, the expression level of anti-PDL1×EGFR rev6 is low.

### Example 7 The activity of anti-PDL1×EGFR bispecific antibody blocking PD1/PD-L1 on cells

PD-1/PD-L1 Blockade Bioassay, Propagation model and method from Promega were used in this study.

PD-L1 aAPC/CHO-K1 cells at logarithmic growth stage were digested by trypsin into single cells, then cells were transferred to white transparent bottom 96-well plate at 100µL/well and 40,000 cells/well, and incubated at 37°C and 5% CO₂ overnight. Anti-PDL1×EGFR bispecific antibody and anti-PD-L1 monoclonal antibody were diluted into 2× working solution concentrations, with an initial concentration of 24nM and were diluted three times step by step. PD1 effector cells with a density of 1.2-4 ×10⁶ cells /mL and cell viability more than 95% were taken and were digested by trypsin into single-cell suspension with 1.25×10⁶ cells/mL. PD-L1 aAPC/CHO-K1 cells planked the day before were taken, the supernatant was discarded, and 40µl of gradient diluted bispecific antibody/PD-L1 monoclonal antibody working solution was added. Then an equal volume of PD1 effector cells was added. The cells were incubated at 37°C, 5% CO₂, for 6 hours. 80µl Bio-Glo detection reagent was added to each well, and the cells were incubated at room temperature for 10 min. Luminescence was read with spectramax i3. The bottom of the plate was covered by an opaque film before reading the plate. Data were analyzed by GraphPad Prism, and IC₅₀ was plotted and calculated.

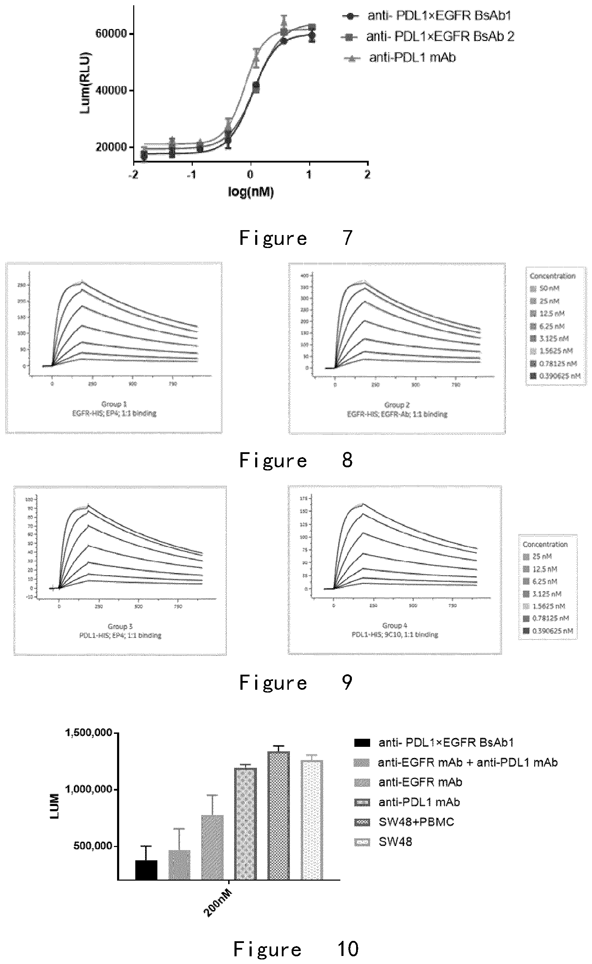

The experimental results are shown in Figure 7. IC₅₀ of anti-PDL1×EGFR BsAb1, anti-PDL1×EGFR BsAb2 and anti-PD-L1 monoclonal antibody is 1.06nM, 1.21nM and 0.78nM, respectively, showing similar effect.

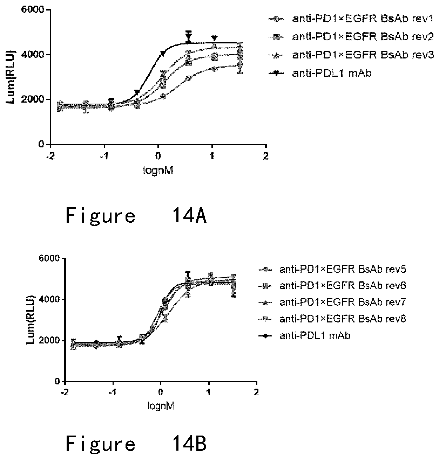

The bispecific antibodies including anti-PDL1×EGFR BsAb rev1, rev2, rev3, rev5, rev6, rev7, and rev8 block the activity of PD1/PD-L1 signaling pathway on cells, and the results are shown in Figure 14. Anti-PDL1×EGFR BsAb rev1, rev2, rev3 and rev7 are significantly worse than the positive control anti-PD-L1 monoclonal antibody (Figure 14A). The activity of anti-PDL1×EGFR BsAb rev5, rev6 and rev8 is not significantly different from that of the positive control anti-PD-L1 monoclonal antibody (Figure 14B).

Based on the results of examples 6 and 7, the ranking of comprehensive performance is anti-PDL1×EGFR BsAb1≥anti-PDL1×EGFR BsAb2 > other bispecific antibodies.

### Example 8: Pharmacokinetic study of anti-PDL1×EGFR bispectic antibody

Five SD rats in each group were taken, which weighed about 200 g. Each rat was injected with anti-PDL1×EGFR BsAb2 at a dose of 3mg via tail vein. Blood collection time points were 0h, 3h, 24h, 48h, 96h, 168h, 336h after administration. Orbital blood was taken, and after blood natural coagulation, serum was taken by centrifugation at 8000 rpm/min.

Serum drug concentration of anti-PDL1 ×EGFR BsAb2 was detected by the following methods:
1) ELISA plate was coated with protein A to detect the Fab segment of the antibody. The plate was coated with protein A and the coating amount was 100ng/well at 4°C overnight. The next day, the plate was washed twice with PBST, and then the plate was blocked at 37°C for 2 hours with PBS+2%BSA. The plate was washed by PBST twice. The standard anti-PDL1×EGFR BsAb2 was diluted starting at 1000ng/mL and 12 gradients were diluted twice step by step. Rat serum samples were diluted 1000-4000 times. The samples from the above two groups were added to the blocked ELISA plate and incubated for 1 hour. After the plates were washed with PBST for two times, HRP labeled mouse anti-human Fab antibody was added and placed at 37°C for 30 min. After the plates were washed with PBST for 3 times, the residual droplets on the plate were patted dry on the absorbent paper as possible. 100µl TMB was added to each hole, and the plate was placed at room temperature (20±5°C) for 5 minutes away from light. The substrate reaction was terminated by adding 50µl 2M H₂SO4 terminating solution to each well, and OD values were read at 450nm Microplate Reader. The calculated half-life is 304 hours.
2) ELISA plate was coated with EGFR-ECD-hFc, 50ng/well. The other methods are the same as above. The calculated half-life is 275 hours.
3) ELISA plate was coated with PDL1-ECD-hFc, 100ng/well. The other methods are the same as above. The calculated half-life is 232 hours.

As shown in Table 1-3, based on the ELISA results of the above three groups, the calculated half-life results are similar, indicating that the analyzed data is reliable.

**Table 1 Results of ELISA plate coated with protein A**

| Experimental group | Half-life (hour) | Maximum concentration (µg/mL) |
|---|---|---|
| 1 | 294.68064 | 194924 |
| 2 | 342.58663 | 169060 |
| 3 | 422.99182 | 189736 |
| 4 | 209.30123 | 207656 |
| 5 | 250.98047 | 204668 |

**Table 2. Results of ELISA plate coated with EGFR-ECD-hFc**

| Experimental group | Half-life (hour) | Maximum concentration (µg/mL) |
|---|---|---|
| 1 | 275.17053 | 315296 |
| 2 | 335.66532 | 238636 |
| 3 | 336.24828 | 265160 |
| 4 | 174.68117 | 310808 |
| 5 | 255.16186 | 278016 |

**Table 3. Results of ELISA plate coated with PDL1-ECD-hFc**

| Experimental group | Half-life (hour) | Maximum concentration (µg/mL) |
|---|---|---|
| 1 | 242.94938 | 213860 |
| 2 | 261.43525 | 194548 |
| 3 | 293.1178 | 225988 |
| 4 | 155.13898 | 220480 |
| 5 | 207.33783 | 225240 |

### Example 9 The affinity of the anti-PDL1×EGFR bispecific antibody to the antigen determined by Biacore 8K

Protein A capture method was used to determine the kinetic parameters of binding of anti-PDL1 ×EGFR bispecific antibody to antigen PDL1-ECD-his. Bispecific antibody with concentration of 2µg/ml was bound to the Protein A chip, and the antigen PDL1-ECD-his was diluted twice step by step from 50nM with 1 ×HBS-EP working solution. Seven concentration gradients were set to bind to the antibody and dissociate in the HBS-EP working solution (as shown in Figure 9).

Protein A capture method was used to determine the kinetic parameters of binding anti-PDL1 ×EGFR bispecific antibody to antigen EGFR-ECD-his. Bispecific antibody with concentration of 2µg/ml was bound to the Protein A chip, and the antigen EGFR-ECD-his was diluted twice step by step from 25nM with 1 ×HBS-EP working solution. Seven concentration gradients were set to bind to the antibody and dissociate in the HBS-EP working solution (as shown in Figure 8).

The kinetic parameters of anti-PDL1 ×EGFR BsAb1 and the control monoclonal antibody binding to EGFR-HIS and PDL1-ECD-his are shown in the following table (Table 4 and Table 5).

The results show that there is no significant difference in the affinity and dissociation constants of the anti-PDL1 ×EGFR bispecific antibody compared with positive control.

**Table 4. Affinity and dissociation constants of antibody and EGFR antigen**

| Group | Analyte 1 Solution | Kinetics Chi² (RU²) | ka (1/Ms) | kd (1/s) | KD (M) |
|---|---|---|---|---|---|
| anti-PDL1×EGFR BsAb1 | EGFR-His | 2.60 E+00 | 6.21 E+05 | 1.33 E-03 | 2.13 E-09 |
| anti-EGFR-mAb | EGFR-His | 6.35 E+00 | 7.92 E+05 | 1.48 E-03 | 1.87 E-09 |

**Table 5. Affinity and dissociation constants of antibody and PD-L1 antigen**

| Group | Analyte 1 Solution | Kinetics Chi² (RU²) | ka (1/Ms) | kd (1/s) | KD (M) |
|---|---|---|---|---|---|
| anti-PDL1×EGFR BsAb1 | PDL 1-His | 2.11 E-01 | 1.48 E+06 | 1.43 E-03 | 9.65 E-10 |
| anti-PDL1 mAb | PDL 1-His | 4.36 E-01 | 1.05 E+06 | 1.30 E-03 | 1.24 E-09 |

### Example 10 Synergistic effect of anti-PDL1×EGFR bispecific antibody at cellular level

Cells were plated in 96-well plates: SW48 cells were cultured to logarithmic growth stage. After digestion with trypsin, the medium was changed into RPMI1640 medium with 5%FBS, and the cell density was adjusted to 100,000 cells/mL. Cells were plated in 96 well plates at 100µL/well, i.e. 10,000 cells/well. The plate was cultured overnight in 5% CO₂ incubator at 37°C.

Next day administration: the concentration of each antibody was 200nM, the human PBMCs were 50,000 cells/well, and the final volume was 200µL/well. The culture was continued in 5%CO₂ incubator at 37°C for seven days.

Reading plate: 7 days after administration, 150µL supernatant was removed with a pipetter, and the remaining supernatant was added into cell titer Glo at 50µL/well, and incubated for 10min. The Luminescence was read, and GraphPad Prism7 was used for data analysis and plotting.

As shown in Figure 10, the killing activity of anti-PDL1 ×EGFR BsAb1 is significantly higher than that of anti-PD-L1 monoclonal antibody and that of anti-EGFR monoclonal antibody. It shows that the anti-PDL1×EGFR BsAb1 plays a synergistic killing effect.

### Example 11 Anti-tumor effect of anti-PDL1×EGFR bispecific antibody in mouse xenograft model

### 1. Anti-tumor effect of anti-PDL1 ×EGFR bispecific antibody on SW48 xenograft model

This experiment was conducted to evaluate the anti-EGFR activity of anti-PDL1×EGFR bispecific antibody in animal models. SW48 colon cancer cell is expressed EGFR on its surferce, and anti-EGFR antibody can effectively inhibit SW48 cell proliferation.

SW48 cells cultured *in vitro* were collected and the cell concentration was adjusted to 3×10⁷ cells /mL in serum-free medium. Under sterile conditions, 100µL cell suspension was inoculated into the subcutaneous in the rib of BALB/C nude mice. The length and width of the xenograft tumor were measured with vernier calipers, and the tumor volume was calculated. The animals were randomly grouped when the tumor grew to 100-200mm³.

The dose of the tested sample anti-PDL1 ×EGFR BsAb1 was 13.3mg/kg, and the dose of positive control anti-EGFR mAb was 10mg/kg. The control group was given the same volume of PBS. The administration method was intraperitoneal administration, with administration volume of 0.2mL/mouse (20g), twice a week, continuously for three weeks.

The xenograft tumor volume was measured twice a week, and the mice were weighed and recorded. The formula for calculating tumor volume (TV) is: TV=1/2× length × width². According to the measured results, the relative tumor volume (RTV) is calculated, and the formula for calculating is as follows: RTV=Vt/V0. Wherein, V0 is the tumor volume measured at the time of separate cage administration (i.e., d0), and Vt is the tumor volume measured at each time. The evaluation index of antitumor activity is relative tumor proliferation rate T/C (%), and the formula for calculating is as follows: T/C (%) = (TRTV/CRTV) ×100 (TRTV: RTV in treatment group; CRTV: RTV in negative control); inhibitory rate =1-T/C (%). Efficacy evaluation criteria: T/C (%)>40% is invalid; T/C (%) ≤40, and after statistical treatment, p≤0.05 is valid.

The experimental results are shown in Figure 11, there is no significant difference between anti-PDL1×EGFR BsAb1 and the positive control anti-EGFR monoclonal antibody.

### 2. Anti-tumor effect of anti-PDL1 ×EGFR bispecific antibody in B-hPD-L1 humanized mouse MC38-hPD-L1 xenograft model

This experiment was conducted to evaluate the anti-PD-L1 activity of anti-PDL1×EGFR bispecific antibody in animal models.

Mouse colon cancer MC38 cells were cultured in a 5%CO₂ incubator at 37°C with DuLbecco's Modified Eagle's (DMEM) medium containing 10% inactivated fetal bovine serum. Human PD-L1 was overexpressed in MC38-hPDL1 cells (genetically modified by Bioxetus) and mouse PD-L1 were knocked out simultaneously. MC38-hPDL1 colon cancer cells suspended with PBS were inoculated subcutaneously into the right side of B-hPD-L1 humanized mice (Bioxetus, C57BL/6 stain) at a concentration of 5×10⁵ cells/0.1mL and a volume of 0.1mL. When the average tumor volume reached about 138mm³, 8 mice with appropriate tumor volume were selected for the experiment.

The dose of anti-PDL1 ×EGFR BsAb1 was 27mg/kg, and the dose of positive control anti-EGFR mAb was 20mg/kg. The control group was given the same volume of PBS. The drug was administered intraperitoneally twice a week for three weeks. The xenograft tumor volume was measured twice a week, and the mice were weighed and recorded.

The experimental results are shown in Figure 12. In the efficacy model of this experiment, compared with the control group (PBS), the tested sample anti-PDL1×EGFR BsAb1 shows significant inhibitory effect on the growth of subcutaneous tumors in B-hPD-L1 humanized mice transplanted with MC38-hPD-L1 cells at the test dose. There is no significant difference compared with positive control anti-EGFR monoclonal antibody.

All references referred to in the present invention are cited as references in this application, just as each reference is cited separately as a reference. In addition, it is understood that after reading the above instruction of the invention, a person skilled in the field may make any alteration or modification to the Invention, and such equivalent forms also fall within the limits of the claims attached to this application.

## Claims

1. An anti-PDL1×EGFR bispecific antibody, comprising two polypeptide chains and two light chains selected from the group consisting of:
(a) The polypeptide chain comprises
VL-PDL1-linker1-VH-PDL1-linker2-VH-EGFR-CH1-CH2-CH3 or
VH-PDL1-linker1-VL-PDL1-linker2-VH-EGFR-CH1-CH2-CH3 or
EGFR-CH1-CH2-CH3-linker2-VL-PDL1-linker1-VH-PDL1 or
EGFR-CH1-CH2-CH3-linker2-VH-PDL1-linker1-VL-PDL1 from the N-terminal to the C-terminal,
the light chain comprises VL-EGFR-CL from the N terminal to the C terminal; or
(b) The polypeptide chain comprises
VL-EGFR-linker1-VH-EGFR-linker2-VH-PDL1-CH1-CH2-CH3 or
VH-EGFR-linker1-VL-EGFR-linker2-VH-PDL1-CH1-CH2-CH3 or
PDL1-CH1-CH2-CH3-linker2-VL-EGFR-linker1-VH-EGFR; or
PDL1-CH1-CH2-CH3-linker2-VH-EGFR-linker1-VL-EGFR from the N terminal to the C terminal,
the light chain comprises VL-PDL1-CL from the N terminal to the C terminal;
wherein, VL-PDL1 is a light chain variable region binding to PD-L1,
the VH-PDL1 is a heavy chain variable region binding to PD-L1,
the VL-EGFR is a light chain variable region binding to EGFR,
the VH-EGFR is a heavy chain variable region binding to EGFR,
the CH1-CH2-CH3 is a heavy chain constant region, the CL is a light chain constant region,
the linker1 and linker2 are each independently flexible peptide linkers,
the VL-PDL1 and the VH-PDL1 form an antigen-binding site that specifically binds to PD-L1,
the VH-EGFR and the VL-EGFR form an antigen-binding site that specifically binds to EGFR.

2. The anti-PDL1×EGFR bispecific antibody of claim 1, comprising:
(a) Two polypeptide chains, wherein the polypeptide chain comprises VL-PDL1-linker1-VH-PDL1-linker2-VH-EGFR-CH1-CH2-CH3 from the N terminal to the C terminal,
wherein,
the VL-PDL1 is a light chain variable region binding to PD-L1;
the linker1 and linker2 are each independently flexible peptide linkers;
the VH-EGFR is a heavy chain variable region binding to EGFR,
the CH1-CH2-CH3 is a heavy chain constant region, and
(b) Two light chains, wherein the light chain comprises VL-EGFR-CL from the N terminal to the C terminal, wherein the VL-EGFR is a light chain variable region binding to EGFR, and the CL is a light chain constant region,
wherein, the VL-PDL1 and the VH-PDL1 form an antigen-binding site that specifically binds to PD-L1,
the VH-EGFR and the VL-EGFR form an antigen-binding site that specifically binds to EGFR.

3. The anti-PDL1×EGFR bispecific antibody of claim 2, comprising:
(a) Two polypeptide chains, wherein the polypeptide chain comprises VL-PDL1-linker1-VH-PDL1-linker2-VH-EGFR-CH1-CH2-CH3 from the N terminal to the C terminal, wherein the VL-PDL1 is a light chain variable region binding to PD-L1, the linker1 is 4 or 5 GGGGS, the VH-PDL1 is a heavy chain variable region binding to PD-L1, the linker2 is 3 or 4 GGGGS, the VH-EGFR is a heavy chain variable region binding to EGFR, the CH1-CH2-CH3 is a heavy chain constant region, and
(b) Two light chains, wherein the light chain comprises VL-EGFR-CL from the N terminal to the C terminal, wherein the VL-EGFR is a light chain variable region binding to EGFR, and the CL is a light chain constant region,
wherein, the VL-PDL1 and the VH-PDL1 form an antigen-binding site that specifically binds to PD-L1,
the VH-EGFR and the VL-EGFR form an antigen-binding site that specifically binds to EGFR.

4. The bispecific antibody of claim 3, wherein the VL-PDL1 comprises light chain CDRs with amino acid sequence as shown in SEQ ID NO: 1-3, the VH-PDL1 comprises heavy chain CDRs with amino acid sequence as shown in SEQ ID NO: 4-6, the VH-EGFR comprises the heavy chain CDRs as shown in SEQ ID NO: 7-9, and the VL-EGFR comprises the light chain CDRs as shown in SEQ ID NO: 10-12.

5. The bispecific antibody of claim 4, wherein the VL-PDL1 has an amino acid sequence as shown in SEQ ID NO: 13, the VH-PDL1 has an amino acid sequence as shown in SEQ ID NO: 14, the VH-EGFR has an amino acid sequence as shown in SEQ ID NO: 15, and the VL-EGFR has an amino acid sequence as shown in SEQ ID NO: 16.

6. The bispecific antibody of any one of claims 1-5, wherein the polypeptide chain has an amino acid sequence as shown in SEQ ID NO: 17 or SEQ ID NO: 18 or SEQ ID NO: 23 or SEQ ID NO: 24 or SEQ ID NO:25, and the light chain has an amino acid sequence as shown in SEQ ID NO: 19;
or the polypeptide chain has an amino acid sequence as shown in SEQ ID NO: 26 or SEQ ID NO: 27 or SEQ ID NO: 28 or SEQ ID NO: 29, and the light chain has an amino acid sequence as shown in SEQ ID NO: 22.

7. The bispecific antibody of any one of claims 1-6, wherein the heavy chain constant region comprises IgG1, IgG2, IgG3 or IgG4 heavy chain constant region, and the light chain constant region comprises κ or λ light chain constant region.

8. An isolated nucleotide, which encodes the bispecific antibody of any one of claims 1-7.

9. An expression vector, which comprises the nucleotides of claim 8.

10. A host cell, which comprises the expression vector of claim 9.

11. A method for producing the bispecific antibody of any one of claims 1-7, which comprises the following steps:
(a) The host cells of claim 10 are cultured under expression conditions to express the bispecific antibody;
(b) Isolation and purification of the bispecific antibody described in (a).

12. A pharmaceutical composition comprising the bispecific antibody of any one of claims 1-7 and a pharmaceutically acceptable carrier.

13. Use of the bispecific antibody of any one of claims 1-7 or the pharmaceutical composition of claim 10 in the preparation of drugs for the treatment of cancer.

14. The use of claim 13, the cancer is selected from the groups consisting of colorectal cancer, non-small cell lung cancer, squamous cell cancer, head and neck cancer, kidney cancer, bladder cancer, ovarian cancer, breast cancer, melanoma, lung cancer, liver cancer, gastric cancer, lymphoma, leukemia, prostate cancer, bone marrow cancer, and other neoplastic malignant diseases.

15. A method for the treatment of cancer, which comprises the step of administrating the bispecific antibody of any one of claims 1-7, or immune conjugate thereof, or the pharmaceutical composition of claims 12, to a subject in need.

16. The method of claim 15, wherein the cancer is selected from the groups consisting of colorectal cancer, non-small cell lung cancer, squamous cell cancer, head and neck cancer, kidney cancer, bladder cancer, ovarian cancer, breast cancer, melanoma, lung cancer, liver cancer, gastric cancer, lymphoma, leukemia, prostate cancer, bone marrow cancer and other neoplastic malignant diseases.

17. An immune conjugate, which comprises:
(a) the bispecific antibodies of any one of claims 1-7; and
(b) a coupling moiety selected from the group consisting of a detectable label, a drug, a toxin, a cytokine, a radionuclide, or an enzyme.

18. Use of the immune conjugate of claim 17for preparing a pharmaceutical composition for the treatment of tumors.
